# EUROPEAN PATENT APPLICATION

(11) **EP 3 207 957 A1**
(43) Date of publication of application: **23.08.2017**
(21) Application number: 16156783.9
(22) Date of filing: 22.02.2016
(51) Int. Cl.: A61N 5/06

(54) **HAIR CARE DEVICE**

(71) Applicant: Koninklijke Philips N.V., 5656 AE Eindhoven (NL)
(72) Inventor: Bremer, Peter, 5656 AE Eindhoven (NL); Zjiroecha, Nikolaj, 5656 AE Eindhoven (NL)
(74) Representative: Steenbeek, Leonardus Johannes

(57) **Abstract**

A nozzle for a hair care device comprises a plurality of diffuser tips for applying IR radiation to a scalp (S) of a user. The diffuser tips have an air outlet (AO) for blowing hair (H) aside to reduce absorption of the IR radiation by the hair (H). Preferably, the diffuser tips each have a light guide (LG) for guiding the IR radiation through the diffuser tip. Preferably, each diffuser tip is provided with an IR LED for generating IR radiation at an intensity not exceeding 15 mW/cm². Preferably, the intensity does not exceed 10 mW/cm². The invention also relates to a hair care device comprising such a nozzle.

## Description

### FIELD OF THE INVENTION

The invention relates to a hair care device.

### BACKGROUND OF THE INVENTION

WO 2011/100711 discloses a hair dryer. A front plate includes a plurality of front plate protrusions and a plurality of front plate vents, and at least one near infrared light emitting diode is associated with an interior portion of the front plate. The near infrared light emitting diodes are adapted to deliver a dose of near infrared light ranging between about 85,000 to about 150,000 micro-Joules/cm²-sec to a target.

### SUMMARY OF THE INVENTION

It is, inter alia, an object of the invention to provide an improved hair care device. The invention is defined by the independent claims. Advantageous embodiments are defined in the dependent claims.

One embodiment of this invention is formed by a hair dryer which applies infrared (IR) radiation to the scalp. The construction is such that at the location where the IR radiation has to be applied, the hairs are moved out of the way by a special construction for blowing aside the hairs temporarily. The invention is based on the recognition that hair absorbs nearly all IR radiation. So there must be as little hair as possible between the IR source and the scalp to increase the biological processes resulting from the IR radiation while preventing deleterious effects (such as heat sensation) from happening.

These and other aspects of the invention will be apparent from and elucidated with reference to the embodiments described hereinafter.

### BRIEF DESCRIPTION OF THE DRAWINGS

Fig. 1 shows an embodiment of a nozzle for a hair care device according to the present invention;
Figs. 2A and 2B illustrate a first embodiment of a diffuser tip for use in the nozzle of Fig. 1; and
Figs. 3A and 3B illustrate a second embodiment of a diffuser tip for use in the nozzle of Fig. 1.

### DESCRIPTION OF EMBODIMENTS

To improve the health of the scalp, which is important as a healthy scalp enables healthy hair, IR radiation which penetrates deep into the skin of the scalp can be applied. A hairdryer can be supplied with IR LEDs having the most effective wavelength for this purpose, basically around 850 nm, but within the range of 780 - 1000 nm.

To get enough effect, the amount of radiation must be sufficient, but also not too high to prevent an unpleasant warm feeling and too much heat penetrating the skin from occurring. For the therapy to be effective and also safe for the scalp skin, a maximum intensity of 15 mW/cm² must be not exceeded. This amount is also expected to be powerful enough, and still safe for the human skin. The risk is that subcutaneous skin tissue is damaged without the user noticing it, since there are very few pain receptors in that layer of skin. Also, the LEDs emit highly concentrated non-visible infrared light which can be hazardous to the human eye if the intensity exceeds 10 mW/cm².

To get a defined amount of IR radiation, it is important to define the distance between IR source and scalp. That can be done by a diffuser with 'fingers' touching the scalp, attached to the hair dryer. The diffuser contains the IR light source and/or enables these IR light sources. The IR light is guided via these 'fingers' to the scalp, while the airflow in and around these fingers is designed in such a way that the hair is blown away from the tips of the 'fingers' allowing the IR light to reach the scalp.

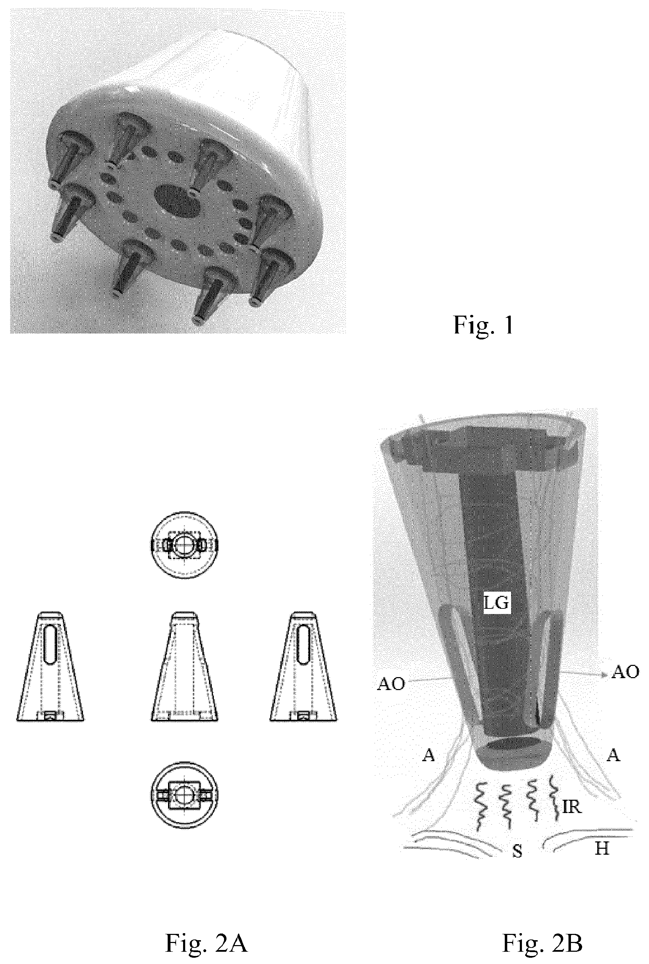

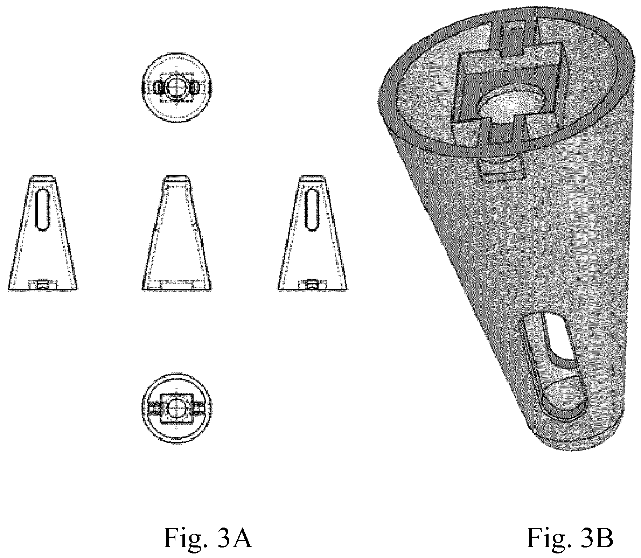

Fig. 1 shows an embodiment of a nozzle for a hair care device according to the present invention. This nozzle can be mounted at the air outlet of a hair dryer in a manner comparable to that shown in Fig. 1 of WO 2011/100711. The nozzle features a plurality of diffuser tips for providing IR radiation. The example of Fig. 1 features 8 diffuser tips; other numbers are alternatively possible. In accordance with an aspect of the present invention, illustrated in more detail in Figs. 2A, 2B and 3A, 3B, these diffuser tips are designed such that in use, hair is blown away so that the IR radiation can reach the scalp.

Figs. 2A and 2B illustrate a first embodiment of a diffuser tip for use in the nozzle of Fig. 1. Fig. 2A illustrates a top view (in use, the top will be closest to the scalp), three side views, and a bottom view (the bottom will be attached to the main body of the nozzle). Fig. 2B illustrates the diffuser tip when viewed from an angle; in Fig. 2B, the bottom of the diffuser tip is shown on top of Fig. 2B, while the top of the diffuser tip is shown at the bottom of Fig. 2B. At the bottom of the diffuser tip, an IR LED can be mounted in a rectangular holder, and air A from the main body of the nozzle enters the diffuser tip. The air A leaves the diffuser tip via slots (air outlets AO) at the sides. In the example of Figs. 2A, 2B, the diffuser tip has 2 air outlets AO at opposite sides of the diffuser tip; other numbers are alternatively possible. As a result of air leaving the sides of the diffuser tip, hair H is blown aside so that the IR radiation that leaves the diffuser tip from the top of the diffuser tip can reach the scalp S. The diffuser tip of Figs. 2A, 2B features a light guide LG that efficiently guides the IR radiation from the IR LED at the bottom of the diffuser tip to the top of the diffuser tip from which the IR radiation leaves the diffuser tip.

Figs. 3A and 3B illustrate a second embodiment of a diffuser tip for use in the nozzle of Fig. 1. The diffuser tip of Figs. 3A, 3B differs from that of Figs. 2A, 2B in that the diffuser tip of Figs. 3A, 3B has no light guide LG. To provide a light guide function, the inner walls of the diffuser tip are reflective, e.g. as a result of making the inner walls white.

In a practical embodiment, LEDs meeting the following specifications appeared to be suitable: wavelength 850-880 nm, 320 mW/sr, 2Θ_{1/2}120°, 3.4 W electrical, 0.8 - 1 W optical, size 7 x 6 mm or smaller, 9 K/W or less, ambient temperature of LED around 40 °C - 50 °C depending on mechanical design. One example of a suitable LED is Osram SFH4235.

It should be noted that the above-mentioned embodiments illustrate rather than limit the invention, and that those skilled in the art will be able to design many alternative embodiments without departing from the scope of the appended claims. While an embodiment of a hair care device is a hair dryer, it suffices that the hair care device is able to generate an air flow to blow hair away; it is not necessary that the air is hot. While in an embodiment, the diffuser tip is provided with an IR LED, in an alternative embodiment the IR LED is present in the main body of the nozzle. In an embodiment, an IR LED in the main body may provide IR light to several diffuser tips by means of light guides. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The mere fact that certain measures are recited in mutually different dependent claims does not indicate that a combination of these measures cannot be used to advantage.

## Claims

1. A nozzle for a hair care device, the nozzle comprising:
a plurality of diffuser tips for applying IR radiation to a scalp (S) of a user, and having an air outlet (AO) for blowing hair (H) aside to reduce absorption of the IR radiation by the hair (H).

2. A nozzle as claimed in claim 1, the diffuser tips each having a light guide (LG) for guiding the IR radiation through the diffuser tip.

3. A nozzle as claimed in any of the preceding claims, each diffuser tip being provided with an IR LED for generating IR radiation at an intensity not exceeding 15 mW/cm².

4. A nozzle as claimed in claim 3, wherein the intensity does not exceed 10 mW/cm².

5. A hair care device comprising a nozzle as claimed in any of the preceding claims.
